# EUROPEAN PATENT APPLICATION

(11) **EP 2 422 818 A2**
(43) Date of publication of application: **29.02.2012**
(21) Application number: 11007013.3
(22) Date of filing: 29.08.2011
(51) Int. Cl.: A61K 47/48, A61P 35/00, A61P 29/00

(54) **Process to produce a diclofenac cyclodextrin conjugate**

(30) Priority: 27.08.2010 PT 00526710
(71) Applicant: TECNIMEDE-SOCIEDADE TECNICO-MEDICINAL, S.A., 2710-089 Sintra (PT)
(72) Inventor: Albuquerque Rocha Gonsalves, Antonio Manuel, 3030-396 Coimbra (PT); de Baptista Veiga, Francisco Jose, 3000-254 Coimbra (PT); Serra, Armenio Coimbra, 3030-185 Coimbra (PT); Fernandes Soares Vieira, Amelia Catarina, 3000 Coimbra (PT); de Albuquerque Carvalho, Rui, 3002-252 Coimbra (PT); de Castro Albuquerque Rocha Gonsalves, Maria A., 3020-255 Coimbra (PT); Ramalho Figueras, Ana Rita, 6200 Covilha (PT)
(74) Representative: Cabral da Cunha Ferreira, Goncalo Maria

(57) **Abstract**

The present invention relates to cyclodextrin conjugate with non-steroidal anti-inflammatory compounds of Formula I, 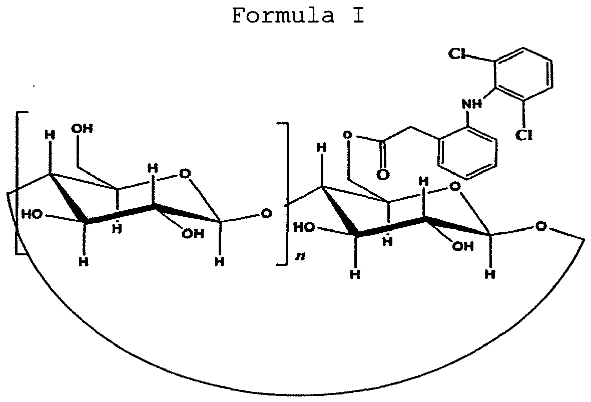 wherein n can be 5, 6 or 7, in particular to a novel diclofenac conjugate.

Further, the present invention also relates to a process to produce said cyclodextrin conjugate by using microwave irradiation.

Such conjugates are particularly useful to the delivery of drugs having anti-inflammatory or anti-tumour activity and therefore, the present invention also provides compositions comprising said conjugates.

## Description

### Field of the invention

The present invention relates to cyclodextrin conjugate with non-steroidal anti-inflammatory compounds, in particular to a novel diclofenac conjugate and to a process to produce said cyclodextrin conjugate by using microwave irradiation. Such conjugates are useful to produce compositions the specific with anti-inflammatory or anti-tumour activity.

### Background of the invention

Cyclodextrins (CDs) are a family of cyclic oligosaccharides consisting of 6-8 glucose units through α-1,4 glycosidic bonds. These macrocyclic carbohydrates with a hydrophilic outer surface and a hydrophobic internal cavity have the ability to interact with poorly water-soluble drugs to form non covalent dynamic inclusion complexes.

The lipophilic microenvironment of the central cavity of CDs gives the ability to form inclusion complexes by taking the whole molecule or rather some non-polar parts in its hydrophobic cavity. Thus, these inclusion complexes are more hydrophilic and consequently have higher water solubility than the free drug increasing the dissolution rate. The increase in the dissolution performance can result in the improvement of the oral bioavailability of class II and IV drugs of BCS (Biopharmaceutical Classification System), improving the pharmacological effect and allowing a reduction in the dose of the administered drug [1-4].

In addition CDs are mainly used for improvement of other desirable properties of drugs such as stability [5], gastrointestinal tolerability [6] and can act as permeability enhancers [7]. It is due to this ability to form complexes that CDs are applicable as functional drug carriers to control rate and/or time profile of oral drug release.

The hydrophilic derivatives (methylated, hydroxyalkylated, branched 6-glucosyl and maltosyl) and ionizable derivatives (carboxyalkyl, carboxymethyl, sulfates, alkylsulfonates) of CDs can serve as potent drug carrier in immediate release and delay release formulations, respectively, while the release rate of water soluble drugs can be retarded by hydrophobic derivatives of CDs (ethylated and peracylated) [8-10].

However, this form of drug carrying as an inclusion complex is not suitable to deliver the drug to the targeted site, because the drug complex can dissociate and the drug is released (because of dilution and/or competitive inclusion effects) before reaching the target organ or tissue to which it is to be delivered.

To overcome this problem the drug can be covalently bound to the cyclodextrin. This kind of conjugate can conveniently be used for site delivery of drugs to the colon [10].

CDs are not absorbed through biological membranes such as the gastrointestinal tract (GIT) because of their bulky and hydrophilic nature. Moreover they are barely hydrolysed in passage through the stomach and small intestine. However, the vast microflora present in the colon, especially Bacteroides, breaks these into small saccharides that are absorbed in the large intestine [11-13].

Therefore, CDs are useful as colon-targeting carriers, and various cyclodextrins pro-drugs have been synthesized in order to obtain site specific delivery of drugs to the colon. In addition, in the USA, natural CDs (α-, β-, γ-) are 'Generally Regarded As Safe' (GRAS) which allows safety assurance of these pro-drugs as new entities [14].

Cyclodextrins conjugates of non-steroidal anti-inflammatory drugs including byphenylacetic acid [15, 16], ketoprofen [17], etodolac [18], mefenamic acid [19], naproxen, flurbiprofen [20], ibuprofen, indomethacin [21]; short-chain fatty acids such as n-butyric acid [22]; steroidal drugs like prednisolone, budesonide [23-25]; a drug for the treatment of inflammatory bowel disease, 5-aminosalicylic acid [26]; β-lactam antibiotics like ampicillin, amoxicillin, cephalexin [27]; and more recently anticancer drug, 5-fluoracil [28], have been designed anticipating new candidates for colon-specific delivery pro-drugs.

Drug molecules were selectively conjugated using the primary or secondary hydroxyl groups of CDs through an ester or amide linkage. Therefore, a limited number of pro-drugs can be formed due to the chemical linkage required in their formation. In the case of prednisolone and budesonide a linker molecule, succinic acid, is used between drug and cyclodextrin, forming esters at both ends of the linker molecule [24, 25].

Modification of β-cyclodextrin with water soluble polymer poly(ethylene glycol) was used to produce conjugates of antibiotics [27] and conjugates of indomethacin and ibuprofen [21]. In order to assess the potential of these conjugates to work as colon targeting pro-drugs, physicochemical properties and drug release behaviour were investigated.

The conventional formulations do not allow the delivery of drugs specifically in the colon due to the absorption and/or degradation of the active substances in the upper GIT by enzymes, the gastric acidity and the strong first passage hepatic effect.

Thus, numerous strategies have been exploited to allow specific drug release in the colon [12, 29]. Among other approaches available to achieve targeted drug release to the colon, prodrugs are designed to pass intact and unabsorbed from the upper GIT and undergo biotransformation in the colon releasing the active drug molecule. This enzymatic biotransformation is carried out by the inherent bacterial flora present in the colon. Thus, this enzyme-trigger mechanism in prodrugs, confer a better colon-target ability which is a new pharmaceutical approach [30-32].

In fact, target drug delivery specifically in the colon has been of great interest due to the importance of this region of the GIT not only for local but also for systemic therapy. The delay in drug absorption may be desired in the treatment of diseases whose symptoms appear mostly in the morning, such as nocturnal asthma, angina and arthritis [33]. Moreover, treatment of colon diseases, including ulcerative colitis, Crohn's disease [34], carcinomas [35] and infections [36] is more effective if the drug release occurs directly in the affected area. Furthermore, the administration of the drug in the site of action not only results in an increase of the therapeutic potency by reduction of the dose administered but also decreases the occurrence of adverse effects, which occur due to the release of drugs in the upper GIT or unnecessary systemic absorption [12, 37].

Non-steroidal anti-inflammatory drugs (NSAIDs) are the most commonly prescribed therapeutic agents [38]. These drugs are widely used as anti-pyretics, analgesics, anti-arthritics. Their anti-inflammatory action is mainly based on inhibition of cyclo-oxygenase (COX), which is an enzyme that converts arachidonic acid into inflammatory mediators such as prostaglandins, prostacyclins, and thromboxanes [39]. Two important COX enzymes have been described: COX-1 which is constitutively expressed in all tissues and makes prostaglandins that protect the stomach and kidney from damage, and COX-2 which is almost undetectable in most tissues under normal physiological conditions, but is induced by inflammatory stimuli, such as cytokines, and produces prostaglandins that contribute to the pain and swelling of inflammation [40].

Beyond these traditional therapeutic indications, nowadays they are also used as agents able to protect against Alzheimer's disease [41, 42]. Other new applications of these drugs are being pursued, particularly in the cure and prevention of adenomas and colon tumours.

Inflammation is now considered a well-established cancer risk factor; a number of inflammatory conditions predispose to cancer, including ulcerative colitis (which increases about 20-fold the risk of developing colon cancer) [43].

NSAIDs are effective chemo-preventive agents against colorectal neoplasia and are considered effective as single agents because they work early in carcinogenesis across multiple pathways [44, 45]. Intervention trials and animal studies indicate that NSAIDs inhibit colorectal carcinogenesis [46]. However, a systemic review examined the benefits and harms of NSAIDs for the prevention of colorectal cancer and adenoma and concluded that the balance of benefits to risks (ulcers and clinically important ulcer complications and cardiovascular harms) does not favour chemoprevention in average risk individuals [47].

Thus, in order to decrease gastrointestinal effects associated with NSAIDs various approaches have been adapted to formulate systems to specific drug release in colon [48-52].

One of the strategies is the synthesis of prodrugs. Several drugs with anti-inflammatory capacity have been combined with the CDs to synthesize prodrugs enabling specific release of drugs in the colon as described above.

Diclofenac [2-(2, 6-dicloranilino) phenyl acetic acid] is a non-steroidal anti-inflammatory drug. This molecule inhibits preferentially COX-2 [53-55], and also inhibits the lipoxygenase pathway, reducing the formation of leucotriens [56]. In addition, diclofenac can inhibit phospholipase A2 which may explain the high anti-inflammatory activity of this drug. This drug is a model drug with great therapeutic potential to be released specifically in the colon not only for its anti-inflammatory capacity and effective chemo-preventive action in colon cancer [57] but also because it is well absorbed in the colon [58]. At the same time the specific release of diclofenac in the colon is of great interest in order to reduce its adverse effects.

Several strategies have been studied with the purpose to release diclofenac in the colon [59-61]. However, until now the conjugation with CDs wasn't explored. On the basis of previous studies, our aim is to conjugate diclofenac with natural CDs. Since, previous studies demonstrated that the ester conjugates have more potential to release drug in colon than amide conjugate [15, 28], the purpose is to synthesize ester conjugates of diclofenac with CDs.

The literature described the synthesis of ester conjugates of cyclodextrin using several methods, including: i) modification of hydroxyl groups of CDs, by an electrophilic reagent, leading to the formation of one group more reactive than the hydroxyl group of cyclodextrin to nucleophilic attack by the drug to the cyclodextrin. The main electrophile reagent used to modify CDs is p-toluenesulphonyl chloride. Tosylated cyclodextrins react with the salt of drug in DMF at 100°C during various hours or days. This approach has been adopted by many authors, particularly in the synthesis of cyclodextrins conjugates with fenilacetic acid [15], etodolac [18], ketoprofen [17], n-butyric acid [22], naproxen, and flurbiprofen [20]; ii) another route to attach a molecule to cyclodextrin consists first in activation of the carboxylic group of drug using carbodiimide derivative, following reaction with cyclodextrin. Carbodiimide derivatives react with the carboxylic group of the drug and allow the formation of an intermediate derivative sufficiently activated to react with the hydroxyl groups of the cyclodextrin. This synthetic route has been used to form conjugates of 5-fluorouracil-1-acetic acid [28], 5-aminosalicylic acid [26] and prednisolone [25] using carbonyldiimidazole as the activating agent. Another carbodiimide derivative, dicyclohexylcarbodiimide, has been used to synthesize conjugate of mefenamic acid [19], ibuprofen, indomethacin [21] and β-lactam antibiotics [27].

In order to overcome the above mentioned drawbacks it is desired to synthesize conjugates between diclofenac and cyclodextrins. Furthermore, it is also desirable to develop a fast, simple and efficient process to synthesize conjugates between diclofenac and cyclodextrins.

### Summary of the invention

It is an object of the present invention to synthesize conjugates between diclofenac and cyclodextrins that may be used to develop drugs with improved anti-inflammatory or anti-tumoral activity.

This object is realised by providing a diclofenac covalently linked to a cyclodextrin, as defined in claim 1.

It is another object of the invention to provide a composition that can be used as a medicament having a specific release activity in the colon.

This object is realised by providing a composition comprising diclofenac and cyclodextrins conjugates, according to claim 5.

A further object of the present invention is to provide a method for making conjugates between diclofenac and cyclodextrins efficiently and conveniently short reaction time.

This object is realised by providing a process for making said conjugates by using a microwave irradiation, as defined in claim 9.

### Description of the drawings

Figure 1 shows a representation of conjugates of cyclodextrins with anti-inflammatory compounds.
Figure 2 - MALDI mass spectrum of compound of diclofenac-β-cyclodextrin conjugate. Fig.2 shows the mass spectra of the diclofenac-β-cyclodextrin-conjugate obtained from microwave conditions after chromatographic isolation. The value of m/z of 1434.308 correspond to the [M+Na] adduct.
Figure 3 shows a representation of sodium diclofenac
Figure 4 - comparison of the 1H-NMR spectra of sodium diclofenac (Figure 4-B) and of the free beta cyclodextrin (Figure 4-A) with the spectrum of the diclofenac-cyclodextrin conjugate (Figure 4-C).

(A) is the ¹H-NMR spectrum of β-cyclodextrin hydrated. (B) is the ¹H-NMR spectrum of sodium diclofenac. (C)is the ¹H-NMR spectrum of diclofenac-β-cyclodextrin conjugate. (D) shows the expansion of (C) ¹H-NMR.

In the spectrum of the diclofenac conjugate, only signals of the diclofenac conjugate are observed which can be assigned to the pure conjugate. Integration of the signals allows concluding that the ratio between cyclodextrin and diclofenac is exactly 1:1.

On the spectrum of sodium diclofenac, the H-2 protons are present at 3.383 ppm. H-5" (6.833 ppm) and H-6" (7.053 ppm) appear as a triplet and H-4" (7.074 ppm) as a duplet. H-5' and H-3' are chemically and magnetically equivalent (7.432 ppm) and appear as a duplet, while H-4' (7.057 ppm) appears as a triplet. In the diclofenac conjugate, all the signals related to diclofenac suffer downfield shifts, except the H-2 protons.

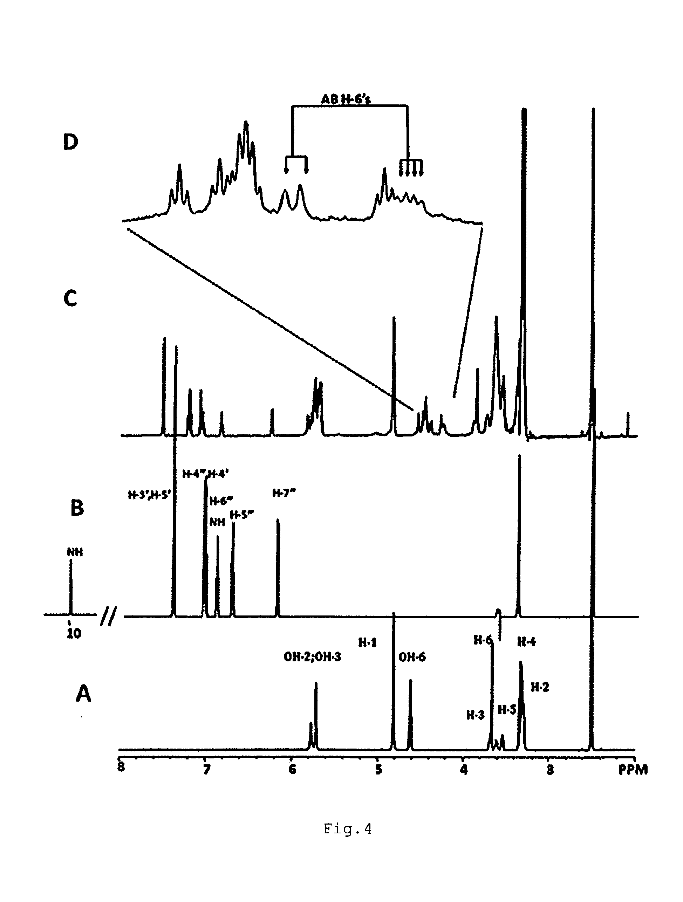

In the expansion (D) of Figure 4 we can observe an AB system (centred at 4.313 ppm). The area of this AB system corresponds to 1/7th of the area of the β-cyclodextrin H-1 protons, indicating again a 1:1 stoichiometric relationship.

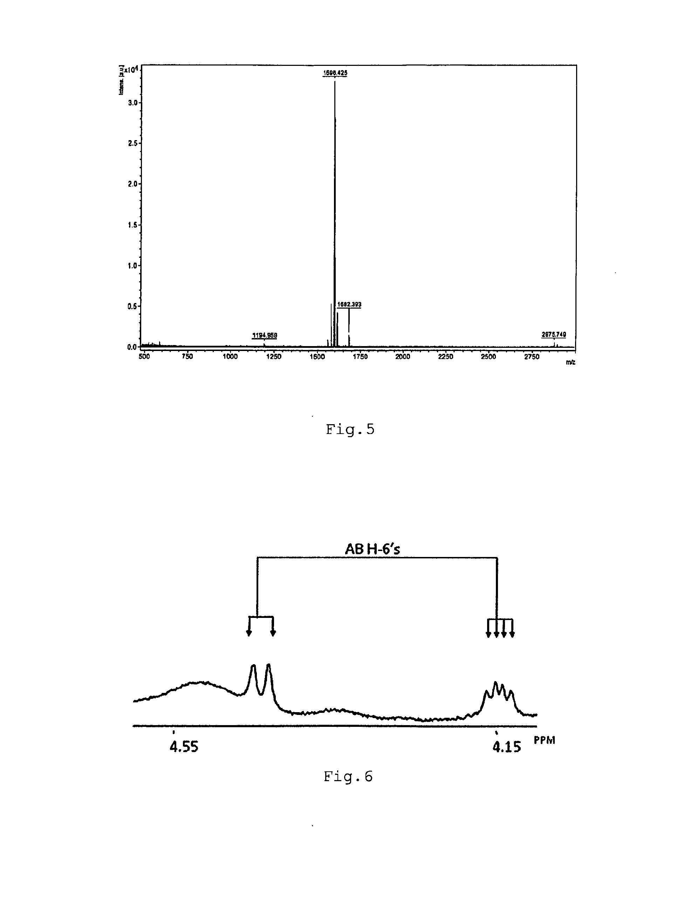

Figure 5: MALDI mass spectrum of compound diclofenac- γ-cyclodextrin conjugate. This figure shows the mass spectra of the diclofenac-γ-cyclodextrin-conjugate obtained from microwave conditions after chromatographic isolation. The value of m/z of 1596.308 correspond to the [M+Na] adduct.

Figure 6: Partial ¹H-NMR spectrum of diclofenac-γ-cyclodextrin conjugate. Fig. 6 shows that the ratio between cyclodextrin and diclofenac is exactly 1:1. In fig. 6 it is possible to observe an AB system centred at 4.308.

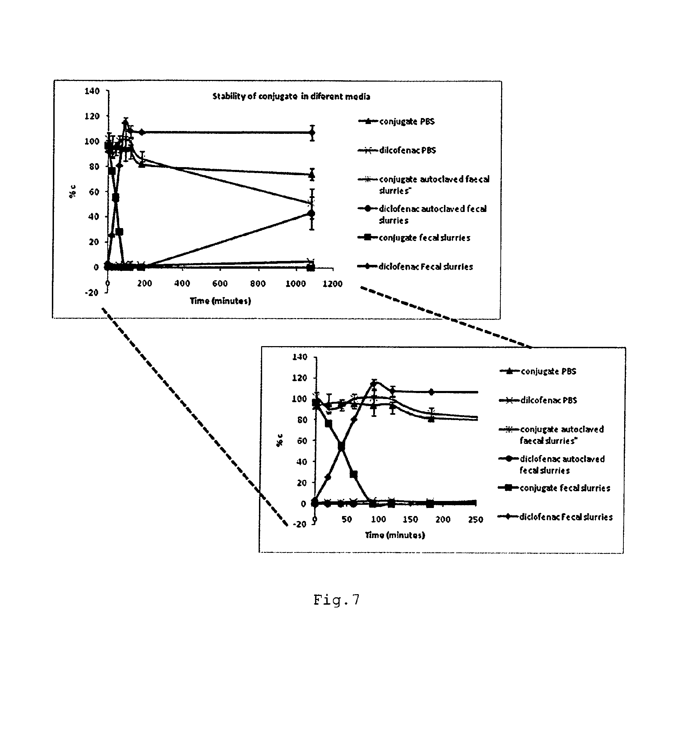

Figure 7: Comparison of stability of conjugate in three different media: faecal slurries, autoclaved faecal slurries and PBS pH=6.8. Fig. 7 shows that the enzymes present in the faecal slurries are able to hydrolyzed the cyclodextrin conjugates and releasing diclofenac at the same time. If slurries are autoclaved the hydrolysis does not occur mainly due to enzyme denaturation.

### Detailed description of the invention

The present invention relates to cyclodextrin conjugate with non-steroidal anti-inflammatory compounds of Formula I, wherein n may be 5, 6 or 7. In particular, the present invention relates to a novel diclofenac conjugate.

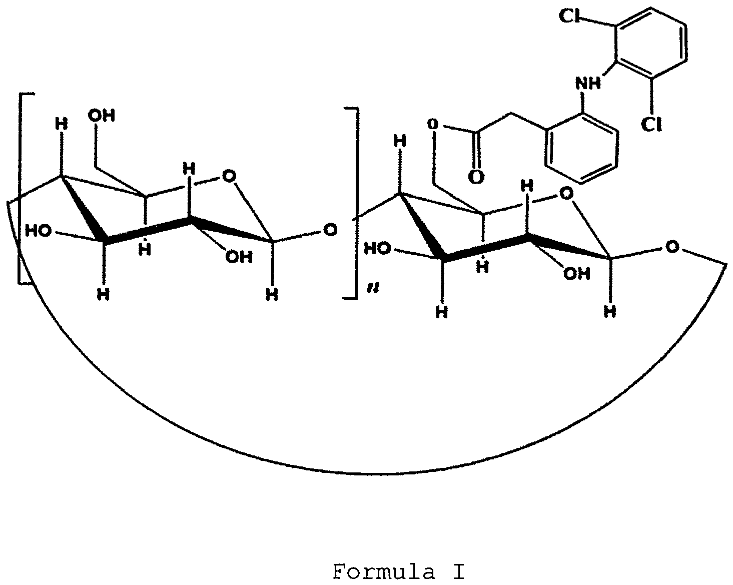

According to the present invention, the diclofenac molecule is covalently linked to a molecule of a cyclodextrin by the formation of a ester bond between the the 6-hydroxyl group of cyclodextrin and the carboxy group of diclofenac.

In a preferred embodiment, the conjugate of the present invention comprises a molecule of β-cyclodextrin or a γ-cyclodextrin. In a most preferred embodiment said conjugate comprises a γ-cyclodextrin molecule. β-cyclodextrin present lower solubility in water than γ-cyclodextrin and therefore drug solubility of conjugates comprising γ-cyclodextrin is higher and preferable.

According to the present invention, α-cyclodextrin is the less preferred molecule amongst the mentioned cyclodextrins due to the associated difficulties for forming stable tosylated conjugates and therefore to have effective reactions with diclofenac.

In a most preferred embodiment the conjugate comprises at least one tosylated cyclodextrin molecule or a mixture of tosylated cyclodextrin molecules, such as mono-tosylated cyclodextrin. The tosylated form of cyclodextrins allows higher rates of conjugates formation by substitution of the tosyl group by the carboxyl group of diclofenac.

The conjugates of the present invention are specifically hydrolized at the colon level and therefore, enzymes present at faecal slurries are able to hydrolize the cyclodextrin conjugates releasing the diclofenac (Fig 7).

Furthermore, said conjugates are very soluble in water. On the other hand, diclofenac is weakly soluble in water.

Therefore, conjugates, according the present invention, provide an adequate way for delivering diclofenac to cells due to the improved solubility rates presented by such molecules.

The conjugates of the invention are useful for the preparation of medicaments for the colon-selective release. Compositions comprising such conjugates may also comprise other agents, such as such as pharmaceutically acceptable vehicles, carriers or excipients. These may include antiadherents, binders, coatings, agents for changing the dissolution rates of active species, disintegrants, fillers, diluents, flavours, colours, lubricants, glidants, preservatives, sorbents and/or sweeteners.

The compositions of the present invention may formulated to be administered through the oral route, in the form of capsules, tablets, protracted-release or enteric-coated forms, prepared using conventional techniques and excipients. Dosages will be of the same order as those known for diclofenac or even lower, due to the improved characteristics of the conjugates described above.

### 1. Characterization of the diclofenac conjugates

### 1.1 Diclofenac-β-cyclodextrin conjugate

The NMR and mass spectrometric analysis indicated that the carboxyl group of diclofenac is covalently bound to one of the primary hydroxyl groups of β-cyclodextrin trough an ester bound.

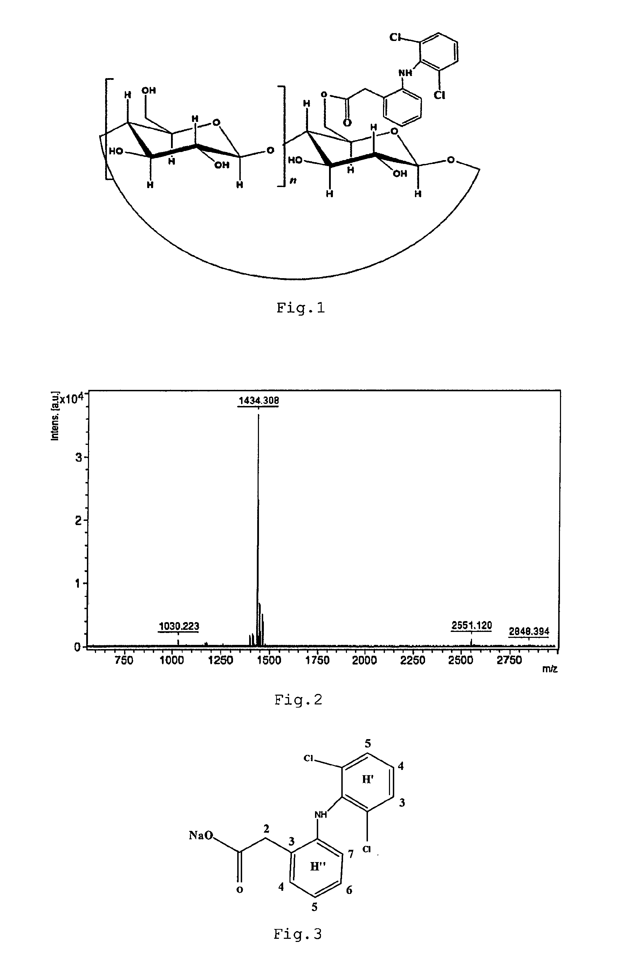

Figure 2 shows the MALDI mass spectra of the diclofenac-β-cyclodextrin-conjugate obtained from microwave conditions after chromatographic isolation. The value of m/z of 1434.308 correspond to the [M+Na] adduct.

The formation of said conjugate was studied by comparison of the 1H-NMR spectra of sodium diclofenac of Formula II, as in Figure 4-B, and of the free beta cyclodextrin according to Figure 4-A, with the spectrum of the diclofenac-cyclodextrin conjugate (Figure 4-C).

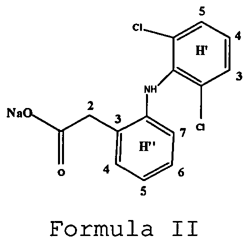

On the spectrum of sodium diclofenac, the H-2 protons are present at 3.383 ppm. The phenyl acetate ring protons are affected by the proximity of two chemical groups, the carboxylic group and the 2,6 - diclorophenyl ring. H-5" (6.833 ppm) and H-6" (7.053 ppm) appear as a triplet and H-4" (7.074 ppm) as a duplet. H-7" is the most shielded because the proximity to the amine group. The signals of 2,6 diclorophenyl ring protons appear downfield. H-5' and H-3' are chemically and magnetically equivalent (7.432 ppm) and appear as a duplet, while H-4' (7.057 ppm) appears as a triplet.

In the diclofenac conjugate, all the signals related to diclofenac suffer downfield shifts, except the H-2 protons (Table 1). These modifications in the chemicals shifts show that the diclofenac is not included in cyclodextrin's cavity, when in the conjugate form. The up-field shift observed for H-2 protons of diclofenac is the result of an ester bond formation.

**Table 1**

| | | | |
|---|---|---|---|
| ¹H Chemical Shifts Corresponding to sodium diclofenac, beta-cyclodextrin hydrate and diclofenac -beta cyclodextrin conjugate in DMSO. | | | |

| **Sodium diclofenac** | **Free δ** | **Conjugate δ** | **Δδ** |
|---|---|---|---|
| H5', H3" (duplet) | 7.432 | 7.520 | 0.088 |
| H4" (duplet) | 7.074 | 7.221 | 0.147 |
| H4' (triplet) | 7.057 | 7.200 | 0.126 |
| H6" (triplet) | 6.920 | 7.053 | 0.133 |
| H5" (triplet) | 6.736 | 6.833 | 0.097 |
| H7" (duplet) | 6.222 | 6.252 | 0.003 |
| H-2 (singlet) | 3.383 | 3.305 | -0.078 |

On the NMR analysis of the unmodified β-cyclodextrin (Figure 4-A), the secondary hydroxyl groups appear downfield (OH-2, singlet 5.765; OH-3, singlet 5.710), because these protons form intramolecular hydrogen bonds that contribute to a deshielding effect. The proton from the primary hydroxyl is a free-rotating proton and thus appears much more shielded, at 4.605 ppm as a triplet. Duplet due to H-1 protons appears at 4.811 ppm. The H-3 (triplet, 3.610 ppm) and H-5 (duplet of triplets, 3.540 ppm) protons are located within the cavity while H-4 (triplet, 3.329 ppm) and H-2 (duplet of duplets, 3.299) which are on the exterior of the cavity.

In the spectrum of the diclofenac conjugate, only signals of the diclofenac conjugate are observed which can be assigned to the pure conjugate. Integration of the signals allows concluding that the ratio between cyclodextrin and diclofenac is exactly 1:1.

Since the protons of the secondary hydroxyl groups of cyclodextrin do not demonstrate a significant shift comparing to the dramatic up-field shift observed in the protons of primary hydroxyl, the ester bond occurs at the hydroxyl of 6-position of cyclodextrin. Moreover, the 6-hydroxyl substitution is also confirmed by the reduction in the integration of the peak due to the hydroxyl protons in 6-position.

The H-6 protons linked to diclofenac resonate downfield relative to the unsubstituted H-6 protons, and present a distinct multiplicity. In the expansion (D) of Figure 4 we can observe an AB system (centred at 4.313 ppm) due to such protons, indicative of a restriction in the mobility upon substitution of the primary hydroxyl group.

The area of this AB system corresponds to 1/7th of the area of the β-cyclodextrin H-1 protons, indicating again a 1:1 stoichiometric relationship.

### 1.2 Diclofenac- γ-cyclodextrin conjugate

The NMR and mass spectrometric analysis indicated that the carboxyl group of diclofenac is covalently bound to one of the primary hydroxyl groups of γ-cyclodextrin trough an ester bound.

Figure 5 shows the mass spectra of the diclofenac-γ-cyclodextrin-conjugate obtained from microwave conditions after chromatographic isolation. The value of m/z of 1596.308 correspond to the [M+Na] adduct.

In the partial ¹H-NMR spectrum of diclofenac-γ-cyclodextrin conjugate (Fig.6) integration of the signals allows observe that ratio between cyclodextrin and diclofenac is exactly 1:1. In this conjugate, similarly to β-cyclodextrin conjugate the ester bond occurs at the hydroxyl of 6-position. The H-6 protons linked to diclofenac resonate downfield relative to the unsubstituted H-6 protons, and present a distinct multiplicity. In figure 6 we can observe an AB system (centred at 4.308) due to such protons.

### 2. Synthesis of cyclodextrin conjugates

Further, the present invention also refers to a process to produce said cyclodextrin conjugate by using microwave irradiation.

Microwave irradiation has been an interesting alternative for heating systems and several chemical reactions. The use of microwaves has progressively emerged as a popular non conventional heating source in the field of organic synthesis largely due to frequent acceleration in reaction rates, improved yields and selectivity.

According to the present invention, cyclodextrin conjugates may be obtained by nucleophylic substitution using microwave irradiation.

Several assays were performed with different conditions of temperature, time and microwave power irradiation, namely at temperatures between 100-200°C, microwave power irradiation varying between 50 to 100 W and during 10 to 60 minutes.

The very good yield rates were obtained at temperature between 130-160°C for 20-40 minutes under focused microwave irradiation with an initial power setting of 75 W, being the best parameter combination the one which combines a temperature 140°C, with a microwave power irradiation set at 75W during 40 minutes.

Therefore, in a preferred embodiment, the process of the invention comprises a reaction between a cyclodextrin molecule and diclofenac by using microwave irradiation at temperatures between 130-160°C for 20-45 minutes under focused microwave irradiation with an initial power setting of 75W.

In a most preferred embodiment, said reaction is performed at a temperature of 160°C, during 40 minutes, under microwave power irradiation of 75W.

For comparative purposes other assays were performed to attempt cyclodextrins conjugates synthesis, namely by the following known processes:
a) activation of drug carboxylic group of diclofenac using a carbodiimide derivatives following reaction with an hydroxyl group of the cyclodextrin;
b) formation of an acid chloride of the diclofenac and reaction with cyclodextrin;
c) modification of cyclodextrins hydroxyl groups, by an electrophilic reagent, leading to the formation of one group more labile to nucleophilic substitution.

However, none of said tested processes allowed formation of the desired conjugate. Only the specific reaction conditions of microwave radiation, according the present invention, with high temperature in a very short reaction time, allow obtaining conjugates as described in the previous section.

Moreover, it was also observed the successful synthesis of the conjugates of the present invention could involve nucleophylic substitution of the tosyl group of cyclodextrins by the carboxyl group of diclofenac. Said cyclodextrins molecule may have more than one tosylated and or comprise a mixture of tosylate forms, such as monotosylates, ditosylates, etc.

Therefore, in a preferred embodiment of the invention the cyclodextrin conjugates are prepared by the reaction between a tosylated cyclodextrin derivative with diclofenac under microwave radiation.

In another preferred embodiment, cyclodextrin molecules comprise more than one tosylated form of cyclodextrin.

In a further preferred embodiment, cyclodextrin molecules comprise a mixture of tosylated cyclodextrins.

### Examples

### Materials:

- β- Cyclodextrin hydrate was purchased from Sigma-Aldrich; γ-cyclodextrin was purchased from Wacker;
- p-toluenesulphonyl chloride was purchased from Sigma-Aldrich;
- sodium diclofenac was kindly provided by Labesfal Genéricos;
- pyridine was dried over potassium hydroxide;
- DMF was dried over sieves prior to use;
- DIAION HP-20 was purchased from Sigma Aldrich.
- Other chemicals and solvents were analytical reagent grade, and deionized water was used.

### Methods:

Reactions were carried out in appropriate 10 mL tick walled glass vials under closed vessel conditions using CEM Discover S-class single mode microwave reactor with temperature, pressure and microwave power monitoring.

Thin layer chromatography (TLC) was conducted out on aluminium plates pre-coated with silica gel and eluted with a mixture of 2-propanol/ethyl acetate/water/ammonia (6:1:3:1) or acetonitrile/water/ammonia (6:3:1).

The visualization of the spots was made as followed: The agent used for spot detection was a mixture composed as follows: p-anisaldehyde (2 mL)/ethanol (36 mL)/acetic acid (5-6 drops)/sulphuric acid (2 mL). The plate was heated to 150°C for 5 min to visualize the spots.

The identity of compounds was confirmed by matrix-assisted laser desorption/ionization (MALDI) spectra and proton nuclear magnetic resonance (1H NMR) spectroscopy.

Mass spectra were performed on a Autoflex III, Bruker using methanol and water as solvents and α-cyano-4-hydroxy-cinnamic acid (HCCA) as matrix.

¹H NMR spectra were acquired on a Varian Unity-500MHz spectrometer, using deuterated dimethyl sulfoxide (DMSO-d6) as solvent and TMS was used as internal reference.

### Example 1: Synthesis of diclofenac- β-cyclodextrin conjugate

β-cyclodextrin tosylated were synthesized following the procedure reported in a previous work [62]. Briefly, at a solution of β cyclodextrin (5 g, 4.34 mmol) in water (110 mL) was added p-toluenesulfonyl chloride (1.25 g, 13.1 mmol) and the result solution was stirred at ambient temperature for 2h under an inert atmosphere.

Aqueous NaOH (2.5 M, 20 mL) was added and the solution was stirred for 10 min before unreacted p-toluenesulfonyl chloride was filtered off. Ammonium chloride (5.8 g) was added to lower the pH to approximately 8. The solution was cooled overnight and the resultant white precipitate was collected by filtration. The white powder was washed with acetone and water and dried under vacuum to afford 10-30 g of the title compound. The product obtained is used directly in the next step.

Sodium diclofenac (105.9 mg, 0.333 mmol) was dissolved in 3 mL anhydrous DMF containing β-cyclodextrin tosylated (429.3 mg) in an appropriate thick-walled glass vial. The reaction vessel was then sealed with a Teflon cap and the reaction mixture is magnetically stirred and heated at temperature varying between 130-160 °C for 20-40 min under focused microwave irradiation with an initial power setting of 75 W. The best conditions are 160°C/ 40 min.

After cooling to room temperature the product was precipitated in acetone. The precipitated was filtered and washed several times with acetone and ethyl ether, filtered off and then dried. Then the product was purified through by DIAION HP-20 ion-exchange chromatography eluting with water/methanol with increasing methanol content. The eluates were monitored by TLC and the conjugate appeared in the eluates of 80% methanol in water. Methanol in the eluate was removed under reduced pressure and the solution was lyophilized. Yield of isolated, pure product: 10-30%.

### Example 2: Synthesis of diclofenac- γ-cyclodextrin conjugate

γ-cyclodextrin tosylated was produced using the modified method described in the literature [63]. In this case, p-toluenesulfonyl chloride (2.02 g, 10.6 mmol) was added to a solution of γ-cyclodextrin (2 g, 1.54 mmol) in pyridine (20 mL) and stirred for 2 h at room temperature under nitrogen atmosphere followed by addition of ethanol (20 mL). After addition of acetone, a solid precipitated and was collected by filtration and washed with acetone and dried and vacuum and at 100°C to afford 1-2g of tosylate. This material can be re-crystallized by dissolving it in isopropanol at boiling temperature and addition of water and then cooling to room temperature, following by storage in a refrigerator overnight.

Sodium diclofenac (79.5 mg, 0.25 mmol) was dissolved in 3 mL anhydrous DMF containing γ-cyclodextrin tosylated (376 mg) in an appropriate thick-walled glass vial. The reaction vessel was then sealed with a Teflon® cap and the reaction misture is magnetically stirred and heated at temperature varying between 130-160 °C for 20-40 min under focused microwave irradiation with an initial power setting of 75 W. The best conditions are 160°C/ 40 min.

After cooling to room temperature the product was precipitated in acetone. The precipitated was filtered and washed several times with acetone and ethyl ether, filtered off and then dried. Then the product was purified through by DIAION HP-20 ion-exchange chromatography eluting with water/methanol with increasing methanol content. The eluates were monitored by TLC and the conjugate appeared in the eluates of 80% methanol in water. Methanol in the eluate was removed under reduced pressure and the solution was lyophilized. Yield of isolated, pure product: 10-30%.

### References

1. Amber Vyas, Shailendra Saraf, and S. Saraf, Cyclodextrin based novel drug delivery systems. Journal of Inclusion Phenomena and Macrocyclic Chemistry 2008. 62: p. 23-42.
2. Marcus E. Brewster and T. Loftsson, Cyclodextrins as pharmaceutical solubilizers. Advanced Drug Delivery Reviews 2007. 59: p. 645-666.
3. Rebecca L. Carrier, Lee A. Miller, and I. Ahmed, The utility of cyclodextrins for enhancing oral bioavailability. Journal of Controlled Release 2007. 123: p. 78-99.
4. Valle, E.M.M.D., Cyclodextrins and their uses: a review. Process Biochemistry, 2004. 39: p. 1033-1046.
5. Rajeswari Challa, et al., Cyclodextrins in Drug Delivery: An Updated Review. AAPS PharmSciTech, 2005. 6 (2).
6. Thorsteinn Loftsson, M.E. Brewster, and M.a. Másson, Role of Cyclodextrins in Improving Oral Drug Delivery. American Journal of Drug Delivery, 2004. 2(4): p. 1-15.
7. Throstein Loftsson, et al., Effects of cyclodextrins on drug delivery through biological membranes. Journal of Pharmaceutical Sciences, 2007. 96(10): p. 2532-2546.
8. F. Hyrayama and k. Uekama, Cyclodextrin-based controlled drug release system. Advanced Drug Delivery Reviews, 1999. 36: p. 125-141.
9. Uekama, K., Recent aspects of Pharmaceutical Application of Cyclodextrins. Journal of Inclusion Phenomena and Macrocyclic Chemistry, 2002. 44: p. 3-7.
10. Uekama, K., Design and Evaluation of Cyclodextrin-Based Drug Formulation. Chemical & Pharmaceutical Bulletin, 2004. 52(8): p. 900-915.
11. V. R. Sinha and R. Kumria, Polysaccharides in colon specific drug delivery. International Journal of Pharmaceutics, 2001. 224: p. 19-38.
12. M. K. Chourasia and S.K. Jain, Pharmaceutical approaches to colon targeted drug delivery systems. Journal of Pharmacy and Pharmaceutical Sciences, 2003. 6(1): p. 33-66.
13. Bernard Flourié, et al., Fate of beta-Cyclodextrinin the Human Intestine. The Journal of Nutrition, 1993. 123: p. 676-680.
14. Astray, G., et al., A review on the use of cyclodextrins in foods. Food Hydrocolloids, 2009. 23(7): p. 1631-1640.
15. Kaneto Uekama, Kunihiro Minami, and F. Hirayama, 6A-O-[(4-Biphenylyl) acetyl]-alpha-, -beta-, and -gamma-cyclodextrins and 6A-Deoxy-6A-[[(4- biphenylyl)acetyl]amino]-alpha-, -beta-, and -gamma-cyclodextrins: Potential Prodrugs for Colon-Specific Delivery. Journal Medicinal Chemistry, 1997. 40: p. 2755-2761.
16. Kunhiro Minami, Fumitoshi Hirayama, and K. Uekama, Colon-Specific Drug Delivery Based on a Cyclodextrin Prodrug: /Release Behavior of Biphenylylacetic Acid from Its Cyclodextrin Conjugates in Rat Intestinal Tracts after Oral Administration. Journal of Pharmaceutical Sciences, 1998 87(6): p. 715-720.
17. Makoto Kamada, et al., Cyclodextrin conjugate-based controlled release system: repeated- and prolonged-releases of ketoprofen after oral administration in rats. Journal of Controlled Release, 2002. 82: p. 407-416.
18. Enrico Rizzarelli, et al., Cyclodextrins functionalised with etodolac as specific site release agents, U.D.S.D. Catania, Editor. 2007: Italy.
19. S. Dev, et al., Synthesis and Pharmacological evaluation of cyclodextrin conjugate prodrug of mefenamic acid. Indian Journal of Pharmaceutical Sciences, 2007. 69(1): p. 69-72.
20. Amal H. El-Kamel, et al., Oral colon targeted delivery systems for treatment of inflammatory bowel diseases: Synthesis, in vitro and in vivo assessment. International Journal of Pharmaceutics, 2008. 248-255.
21. Hassan Namazi, Seifali Bahrami, and A.A. Entezami, Synthesis and Controlled Release of Biocompatible Prodrugs of β-Cyclodextrin Linked with PEG Containing Ibuprofen or Indomethacin. Iranian Polymer Journal, 2005. 14 (10): p. 921-927.
22. F. Hirayma, et al., Release Characteristics of a Short-Chain Fatty Acid,n-Butyric Acid, from Its b-Cyclodextrin Ester Conjugate in Rat Biological Media. Journal of Pharmaceutical Sciences, 2000. 89(11): p. 1486-1495.
23. Hideki Yano, et al., Colon-specific delivery of prednisolone-appended alpha-cyclodextrin conjugate: alleviation of systemic side effect after oral administration. Journal of Controlled Release, 2002. 79: p. 103-112.
24. Prabhakara Prabhu, et al., Synthesis and Investigation of Colon Specific Polymeric Prodrug of Budesonide with Cyclodextrin. Indian Journal of Pharmaceutical Education Research, 2009. 43(3): p. 295-299.
25. Hideki Yano, et al., Preparation of Prednisolone-Appended alpha-, beta- and gamma-Cyclodextrins: Substitution at Secondary Hydroxyl Groups and In Vitro Hydrolysis Behavior. Journal of Pharmaceutical Sciences, 2001. 90(4).
26. Meijuan Zou, et al., Synthesis and properties of polysaccharide prodrugs of 5-aminosalicylic /acid as potential colon-specific delivery system. European Journal of Pharmaceutics and Biopharmaceutics, 2005. 59: p. 155-160.
27. Hassan Namazi and A. Kanani, Synthesis of new prodrugs based on β-CD as the natural compounds containing b-lactam antibiotics. Journal of Bioactive and Compatible Polymers 2007. 22: p. 77-88.
28. Koichi Udo, et al., 5Fluorouracil acetic acid/beta cyclodextrin conjugates: Drug release behavior in enzymatic and rat cecal media International Journal of Pharmaceutics, *in press.*
29. Basit, A.W., Advances in colonic drug delivery. Drugs, 2005. 65(14): p. 1991-2007.
30. Yunjin Jung and Y.M. Kim, What should be considered on design of a colon-specific prodrug? Expert Opinion on Drug Delivery, 2010. 7(2): p. 245-258.
31. Sinha, V.R. and R. Kumria, Polysaccharide matrices for microbially triggered drug delivery to the colon. Drug Developement Industrial Pharmacy, 2004. 30(2): p. 143-50.
32. Sinha, V.R. and R. Kumria, Colonic drug delivery: prodrug approach. Pharmaceutical Research, 2001. 18(5): p. 557-64.
33. Schellekens, R.C., et al., Pulsatile drug delivery to ileo-colonic segments by structured incorporation of disintegrants in pH-responsive polymer coatings. Journal of Control Release, 2008. 132(2): p. 91-8.
34. Meissner, Y. and A. Lamprecht, Alternative drug delivery approaches for the therapy of inflammatory bowel disease. Journal of Pharmaceutical Sciences, 2008. 97(8): p. 2878-91.
35. Lamprecht, A., et al., Microsphere design for the colonic delivery of 5-fluorouracil. Journal of Controlled Release, 2003. 90(3): p. 313-22.
36. Raghavendra C. Mundargi, et al., Development of Polysaccharide-Based Colon Targeted Drug Delivery Systems for the Treatment of Amoebiasis. Drug Development and Industrial Pharmacy, 2007. 33: p. 255-264.
37. Gupta, H., D. Bhandari, and A. Sharma, Recent trends in oral drug delivery: a review. Recent Patents on Drug Delivery Formulation, 2009. 3(2): p. 162-73.
38. Jones, R., Nonsteroidal anti-inflammatory drug prescribing: past, present, and future. The American Journal of Medicine, 2001. 110(1, Supplement 1): p. S4-S7.
39. Kim, K., et al., The use of corticosteroids and nonsteroidal antiinflammatory medication for the management of pain and inflammation after third molar surgery: A review of the literature. Oral Surgery, Oral Medicine, Oral Pathology, Oral Radiology, and Endodontology, 2009. 107(5): p. 630-640.
40. J. R. Vane and R.M. Botting, Anti-inflammatory drugs and their mechanism of action. Inflammation Research, 1998. 47(Supplement 2): p. S78-S87.
41. in t' Veld, B.A., et al., Nonsteroidal antiinflammatory drugs and the risk of Alzheimer's disease. New England Journal of Medicine, 2001. 345(21): p. 1515-21.
42. Etminan, M., S. Gill, and A. Samii, Effect of non-steroidal anti-inflammatory drugs on risk of Alzheimer's disease: systematic review and meta-analysis of observational studies. British Medical Journal, 2003. 327(7407): p. 128.
43. Coussens, L.M. and Z. Werb, Inflammation and cancer. Nature, 2002. 420(6917): p. 860-7.
44. Cuzick, J., et al., Aspirin and non-steroidal anti-inflammatory drugs for cancer prevention: an international consensus statement. Lancet Oncology, 2009. 10(5): p. 501-7.
45. Ulrich, C.M., J. Bigler, and J.D. Potter, Non-steroidal anti-inflammatory drugs for cancer prevention: promise, perils and pharmacogenetics. Nature Reviews Cancer, 2006. 6(2): p. 130-40.
46. E., G., The prevention of colorectal cancer by aspirin use. 1999. 53(7): p. 303-308.
47. Rostom, A., et al., Nonsteroidal anti-inflammatory drugs and cyclooxygenase-2 inhibitors for primary prevention of colorectal cancer: a systematic review prepared for the U.S. Preventive Services Task Force. Annals of Internal Medicine, 2007. 146(5): p. 376-89.
48. Luppi, B., et al., pH-sensitive polymeric physical-mixture for possible site-specific delivery of ibuprofen. European Journal of Pharmaceutics and Biopharmaceutics, 2003. 55(2): p. 199-202.
49. Piao, Z.-Z., M.-K. Lee, and B.-J. Lee, Colonic release and reduced intestinal tissue damage of coated tablets containing naproxen inclusion complex. International Journal of Pharmaceutics, 2008. 350(1-2): p. 205-211.
50. Orlu, M., E. Cevher, and A. Araman, Design and evaluation of colon specific drug delivery system containing flurbiprofen microsponges. International Journal of Pharmaceutics, 2006. 318(1-2): p. 103-117.
51. Maestrelli, F., et al., Microspheres for colonic delivery of ketoprofen-hydroxypropyl-[beta]-cyclodextrin complex. European Journal of Pharmaceutical Sciences, 2008. 34(1): p. 1-11.
52. Akhgari, A., et al., Statistical optimization of indomethacin pellets coated with pH-dependent methacrylic polymers for possible colonic drug delivery. International Journal of Pharmaceutics, 2005. 305(1-2): p. 22-30.
53. Timothy D. Warner, et al., Nonsteroid drug selectivities for cyclo-oxygenase-1 rather than cyclo-oxygenase-2 are associated with human gastrointestinal toxicity: A full in vitro analysis. Proceedings of the National Academy of Sciences, 1999. 96(13): p. 7563-7568.
54. Yamazaki, T., et al., Diclofenac, a non-steroidal anti-inflammatory drug, suppresses apoptosis induced by endoplasmic reticulum stresses by inhibiting caspase signaling. Neuropharmacology, 2006. 50(5): p. 558-567.
55. Hinz, B., et al., Aceclofenac spares cyclooxygenase 1 as a result of limited but sustained biotransformation to diclofenac. Clinical Pharmacology & Therapeutics, 2003. 74(3): p. 222-235.
56. D J Elder, A.H., D E Halton, and C. Paraskeva, Indication of apoptotic cell death in human colorectal carcinoma cell lines by a cyclooxygenase-2 (COX-2)-selective nonsteroidal anti-inflammatory drug: independence from COX-2 protein expression. Clinical Cancer Research, 1997 3: p. 1679-1683.
57. Kaur Saini, M., et al., Chemopreventive response of diclofenac, a non-steroidal anti-inflammatory drug in experimental carcinogenesis. Nutrición Hospitalaria, 2009. 24(6): p. 717-23.
58. C.H. Gleiter, et al., Colonoscopy in the investigation of drug absorption in healthy volunteers. Gastrointestinal endoscopy 1985. 31(2): p. 71-73.
59. Gonzalez-Rodriguez, M.L., et al., In vitro release of sodium diclofenac from a central core matrix tablet aimed for colonic drug delivery. European Journal of Pharmaceutical Sciences, 2003. 20(1): p. 125-31.
60. Ambrogi, V., et al., Eudragit® and hydrotalcite-like anionic clay composite system for diclofenac colonic delivery. Microporous and Mesoporous Materials, 2008. 115(3): p. 405-415.
61. Cheng, G., et al., Time- and pH-dependent colon-specific drug delivery for orally administered diclofenac sodium and 5-aminosalicylic acid. World Journal Gastroenterologyl, 2004. 10(12): p. 1769-74.
62. Michael McNaughton, et al., Cyclodextrin-Derived Diorganyl Tellurides as Glutathione Peroxidase Mimics and Inhibitors of Thioredoxin Reductase and Cancer Cell Growth. Journal of Medicinal Chemistry, 2004. 47(1): p. 233-239.
63. Kahee Fujita, et al., Synthesis of 6A,6X-di-O-(p-tosyl)-.gamma.-cyclodextrins and their structural determination through enzymic hydrolysis of 3A,6A;3X,6X-dianhydro-.gamma.-cyclodextrins. The Journal of Organic Chemistry, 1988. 53(9): p. 1943-1947.

## Claims

1. A compound of Formula I 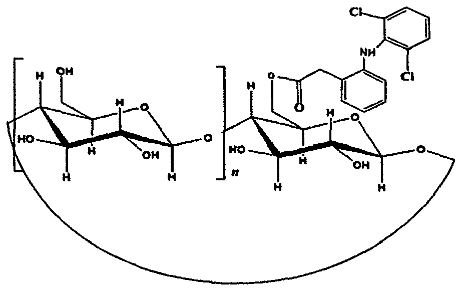 comprising a covalent linked diclofenac molecule to a molecule of a cyclodextrin, wherein n is 5, 6 or 7.

2. A compound, according to the previous claim, wherein the cyclodextrin molecule is a β- cyclodextrin or a γ-cyclodextrin.

3. A compound, as described in any of the previous claims, for use as a medicament.

4. A compound, according to the previous claim, for use as anti-inflammatory and/or anti-tumour drug.

5. A composition comprising the compounds as described in any of the claims 1 or 2.

6. A composition, according to the previous claim, comprising other compounds such as pharmaceutically acceptable vehicles, carriers or excipients.

7. A composition, according to any of the claims 5 or 6, for use as a medicament.

8. A composition, according to any of the claims 5 or 6, for use as anti-inflammatory and/or anti-tumour drug.

9. A process for synthesize a conjugate compound of Formula
I, 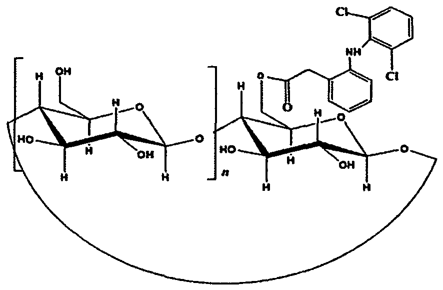 comprising a covalent linked diclofenac molecule to a molecule of a cyclodextrin, wherein n is 5, 6 or 7.

10. A process, according to the previous claim, wherein a cyclodextrin molecule reacts with a diclofenac molecule under microwave irradiation.

11. A process, according to claim 10, wherein at least one cyclodextrin molecule is tosylated.

12. A process, according to the previous claim, wherein cyclodextrins comprise more than one tosylated form of cyclodextrin.

13. A process, according to any of the claims 11 or 12, wherein cyclodextrins comprise a mixture of tosylated cyclodextrins.

14. A process, according to claim 10, wherein the temperature varies between 130-160°C for 20-40 minutes under focused microwave irradiation with an initial power setting of 75W.

15. A process, according to claim 14, wherein the reaction occurs at a temperature of 140°C, during 40 minutes under microwave power irradiation of 75W.
